# EUROPEAN PATENT SPECIFICATION

(11) **EP 2 424 502 B1**
(45) Date of publication and mention of the grant of the patent: **28.03.2018**
(21) Application number: 10723331.4
(22) Date of filing: 27.04.2010
(51) Int. Cl.: A61K 9/00, A61K 9/16, A61K 9/20, A61K 9/28, A61K 31/07, A61P 27/02, A61P 27/06, A61P 27/12, A23L 33/105, A23P 20/10, A23G 1/54

(54) **COMPOSITIONS CONTAINING A MIXTURE OF LUTEIN AND ZEAXANTHINE AND COATED WITH CHOCOLATE**
ZUSAMMENSETZUNGEN AUS EINEM GEMISCH VON LUTEIN UND ZEAXANTHIN UND MIT EINEM SCHOKOLADENÜBERZUG
COMPOSITIONS CONTENANT UN MÉLANGE DE LUTÉINE ET DE ZÉAXANTHINE ET RECOUVERTES DE CHOCOLAT

(30) Priority: 29.04.2009 IT MI20090732
(43) Date of publication of application: 07.03.2012
(73) Proprietor: Graal Srl, 20900 Monza (MB) (IT)
(72) Inventor: SENECI, Alessandro, I-20090 Segrate MI2 (MI) (IT); SENECI, Antonio, I-20100 Milano (IT)
(74) Representative: Pistolesi, Roberto
(86) International application number: PCT/IB2010/051828
(87) International publication number: WO 2010/125516

(56) References cited:
- WO-A-03/063848
- WO-A-2007/076416
- WO-A2-2009/037562
- US-A- 4 271 142
- US-A- 5 102 664
- US-A1- 2005 249 821
- US-A1- 2006 270 739

## Description

Lutein and Zeaxanthin are two carotenoids, i.e. natural pigments, liposoluble derivatives of xanthophyll.

Lutein and zeaxanthin have identical chemical formulae and they are isomers, but not stereoisomers, with respect to each other.

The main difference between these two molecules is the position of a double bond in one of the two terminal rings; this difference leads to lutein having three chiral centres while zeaxanthin has two. The main structure formula of zeaxanthin is (3R, 3'R)-β,β-carotene-3,3'-diol (zeaxanthin), while the main structure formula of lutein is (3R, 3'R, 6'R)-β,ε-carotene-3,3'-diol (lutein).

Lutein and zeaxanthin are extracted from petals of *Tagetes erecta* (*Marigold flower* or Indian rose), an excellent source of these carotenoids. Extraction allows obtaining a mixture of lutein and zeaxanthin esterified with fatty acids, such as for example lauric, myristic and palmitic acid. Lutein and zeaxanthin are obtained in free form through saponification of two esterified carotenoids.

Extraction and purification of the two carotenoids from the petals of *Tagetes erecta* leads to a percentage of about 94% of lutein and about 1.5% of zeaxanthin; the remainder is made up of traces of other isomers of lutein and zeaxanthin.

Lutein and zeaxanthin are also present in nature in dark-leaved vegetables, in fruits, in wheat or in the egg yolk while they are present in the organism mainly in the eye, in particular in a small portion of the retina responsible for central vision called macula.

The role of lutein and zeaxanthin from a physiological point of view is that of protecting the retina against oxidative stress and against the so-called "high energy" light (i.e. the blue light of the visible spectrum).

As a matter of fact, from a metabolic point of view the retina is the most active tissue of the entire organism and, hence, it is the most exposed to oxidative risk and formation of free radicals.

Several studies have revealed a close relation between the concentration of lutein and zeaxanthin in the macula and the pigmentation of the eye, leading to the observation that the higher the level of pigmentation, the lower the risk of diseases correlated to the eye. (Stringham et al "Macular pigment and visual performance under glare conditions" -Optom Vis. Sci.; 2008; Wooten and Hammond et al. "Macular pigment: influences on visual acuity and visibility" -Prog. Retin. Eye Res.; 2002;)

Lutein and zeaxanthin are extremely liposoluble molecules and, in the organism, they bind with liprotein molecules which carry them in the various regions. Scientific studies have revealed that 52% of lutein and 44% of zeaxanthin are carried by HDL, while only 22% of both substances binds with LDL while other carotenoids are carried - 20-25% - by HDL and - 50-57% - by LDL.

From the blood flow, lutein and zeaxanthin are distributed in various organs such as, for example, liver, breast, colon, uterine cervix and thus in particular in the eye.

The mechanism through which lutein and zeaxanthin are incorporated in the retina is not yet clearly known.

Currently available in the market are preparations containing lutein and zeaxanthin formulated in form of swallowable tablet or capsule, gastro-protected or non-gastro-protected, with gastric or intestinal absorption. However, given that these molecules are extremely hydrophobic, they are usually characterised by a difficult absorption.

As raw materials, lutein and zeaxanthin are currently available in the market, in free or esterified form, as mixture thereof in form of dispersible powder, non-dispersible powder, oily suspension, aqueous suspension or microcapsules.

Therefore, there arises the need of providing new compositions based on lutein and zeaxanthin with improved absorption, thus capable of carrying a higher amount of said substances in circulation, preferably at ocular level.

WO2009/037562 discloses a highly palatable chocolate based nutritional supplement formulation comprising lutein, zeaxanthin, lecithin and other active ingredients such as vitamins, fatty acids and antioxidants, which may be useful for the treatment or prevention of macular degeneration.

Now, it has surprisingly been discovered that such absorption difficulties may be overcome through the formulation of lutein and zeaxanthin in orobuccal absorption compositions.

Lutein and zeaxanthin are administered and absorbed directly in the oral cavity through such specific compositions, generating a much quicker absorption and greater bioavailability with respect to the current methods of administration using tablets, both gastro-resistant and non-gastro-resistant, considering the same dose of the active ingredient.

A similar technical effect is interpretable in the light of the fact that the oral haematic system drains in the superior vena cava avoiding the portal venous system responsible for the known first pass effect, improving the systemic bioavailability.

Oral absorption of lutein and zeaxanthin is essential for such active ingredients to reach the retina, preferably in the macula, without being processed by the liver where their functionality would be considerably reduced.

In addition, a similar improved oral absorption allows preventing the hydrolytic action of the acids at gastric level to which lutein and zeaxanthin are extremely sensitive, when formulated in swallowable non-gastro-protected compositions.

Furthermore, the rapid bioavailability obtained from oral absorption allows an improved protective effect, in particular with respect to glare conditions.

Thus, object of the present invention are orobuccal compositions in form of tablets, capsules, granules or pastilles containing a mixture of lutein and zeaxanthin and at least one physiologically acceptable excipient, characterised in that they comprise an outer coating containing chocolate.

Said mixture of lutein and zeaxanthin is thus intended as a mixture of lutein and zeaxanthin alongside one or more common excipients, in one of the aforedescribed forms available in the market (dispersible powder, non-dispersible powder, oily suspension, aqueous suspension or microcapsules).

The orobuccal compositions of the invention are preferably characterised in that they have high palatability, which guarantees easy oral administration thereof.

Said high palatability also determines - in the treated subject (human or animal) - longer period of permanence of the composition in the oral cavity, and ensuing lower impulse to swallow, which contributes to improve the speed of absorption and the bioavailability of the active ingredient.

In addition, said high palatability represents a considerable advantage in paediatric administration. As a matter of fact, children do not like the normal pharmaceutical forms and known are problems related to intake of solid pharmaceutical forms at paediatric age.

The compositions according to the present invention may thus be administered to human beings, intended both as adult subjects and as "paediatric population", where the term "paediatric population" is used to indicate the part of the population ranging between birth and eighteen years of age.

The mixture of lutein and zeaxanthin is present in the compositions of the invention at an amount preferably comprised between 0.1% and 5%, more preferably between 0.15% and 4.5% in weight, with respect to the total weight of the compositions. The abovementioned intervals are calculated as a percentage of the mixture of lutein and zeaxanthin, not having the necessary excipients in commercial form.

The orobuccal compositions of the invention are formulated in form of tablet, capsule, granules or pastille, preferably in form of coated tablet. The compositions of the invention may also be formulated in sublingual and/or effervescence form, preferably sublingual and/or effervescent tablet or granule.

The term "orobuccal" or "buccal" according to the invention refers to compositions capable of dissolving and immediately releasing the active ingredient contained therein upon contact with the oral mucosa. In this manner, the active ingredient may be directly absorbed in the oral mucosa, thus avoiding the hepatic system.

According to the invention, the term "sublingual" according to the invention refers to a particular orobuccal pharmaceutical form which allows absorption in the lower part of the buccal mucosa, an extremely moist portion. The sublingual mucosa is richly vascularised by the ranine veins which make the zone ideal for the absorption of active ingredients (maximum absorption capacity of all oral tissues).

According to the invention the sublingual compositions may be slightly effervescent and non-effervescent tablets or granules.

In order to obtain the orobuccal form, the compositions of the invention are preferably formulated using the following classes of excipients: surfactants (cationic, anionic or non-ionic), diluents, aggregants or binding agents, lubricants, glidants, disaggregants, solubilisers, emulsifiers, sweeteners, flavouring agents, pH regulators or their mixture .

More preferably, the orobuccal compositions according to the invention are formulated with silica gel or dispersed colloidal silica, soy lecithin, polysorbates, sodium lauryl sulfate, phospholipids, sodium carbonate, sodium bicarbonate, mineral bases (for example aluminium hydroxide, magnesium silicate aluminium, magnesium hydroxide carbonate, tribasic magnesium phosphate, magnesium silicate, magnesium peroxide), cocoa paste, cocoa powder, cocoa butter or a mixture thereof.

According to a preferred embodiment, the compositions of the invention contain at least one emulsifier, at least one surfactant or a mixture thereof.

Preferably, said at least one emulsifier is soy lecithin, while said surfactant is preferably selected from among polysorbate 80 (Tween 80) and/or sodium lauryl sulphate. More preferably, the compositions of the invention contain a mixture of soy lecithin and polysorbate 80.

The presence of at least one emulsifier, at least one surfactant or their mixture in the compositions of the invention leads to an improved cellular absorption of the mixture of lutein and zeaxanthin.

In particular, in order to obtain the orobuccal form said excipients are present in the compositions of the invention at an amount varying between 0.3 and 80% in weight, preferably between 0.5 and 60% in weight, with respect to the total weight of the composition.

According to the invention, the term "effervescent" instead refers to compositions capable of developing carbon dioxide when at contact with water and/or with the buccal environment, in the presence of saliva.

In order to obtain the effervescent form, the compositions of the invention are preferably formulated using dicarboxylic and tricarboxylic acids or a mixture thereof.

More preferably, the effervescent compositions according to the invention are formulated with citric acid, tartaric acid, adipic acid, monosodium phosphate, alginic acid or their mixture - for the acid part - and magnesium hydroxycarbonate, sodium or potassium bicarbonate or a mixture thereof, for developing carbon dioxide.

In case of slightly effervescent sublingual tablets, an amount of citric acid shall be added to obtain the effervescence at moderate amounts (slightly effervescent). In particular, said citric acid is added into the slightly effervescent sublingual tablets at an amount varying between 5% and 40%, preferably between 7% and 35% in weight, with respect to the total weight of the composition.

In particular, in order to obtain the effervescent form said excipients are present in the compositions of the invention at an amount varying between 10% and 80% in weight, with respect to the total weight of the composition, preferably between 15% and 75% in weight.

The compositions of the invention may thus be formulated in orobuccal, sublingual or effervescent form and in a combined orobuccal and effervescent form or sublingual and effervescent form.

Further useful excipients according to the invention are selected from among sodium citrate, calcium citrate, magnesium citrate, potassium citrate, sodium phosphate, calcium phosphate, magnesium phosphate, potassium phosphate, light magnesium oxide, magnesium hydroxide, magnesium hydroxycarbonate, sodium carbonate, sodium chloride, potassium carbonate, sodium bicarbonate, potassium bicarbonate, sodium aspartate, calcium ascorbate, ascorbic acid, stearic acid and its salts, oleic acid, 1-leucine, glycerol behenate, hydroxypropyl methylcellulose, crosspovidone, hydrogenated vegetable oil (such as for example palm oil), palm butter, xylitol, maltitol, sorbitol, mannitol, sucralose, acesulfame K, cyclamate sodium, aspartame, sucrose, fructose, dextrose, maltose, spray dried malt, sodium aspartate, neohesperidine, maltodextrines, inositol, inulin, beer yeast, silica gel, vegetable fibres (such as for example pea fibre), chitosan, flavouring agents (essential oils, powders and the like): citrus extract, mint (peppermint, spearmint, sweet mint), badian anethole, vanilla, sage, liver, sodium glutamate, fish meal, chicken, grapefruit, peach, lime, chocolate, bread, almond, milk powder, soy, lyophilised or dried fruits.

These excipients are used in concentrations ranging between 0.3% and 80%, preferably between 0.5% and 60%, with respect to the total weight of the composition

The compositions of the invention may also contain one or more active ingredients. Such one or more additional active ingredients are preferably contained in the compositions of the invention at an amount varying between 1% and 45% in weight, more preferably between 1.5% and 40%, with respect to the total weight of the composition.

Such additional active ingredients are preferably selected from among short chain fatty acids, medium chain fatty acids, vitamin C, vitamin D, vitamins of group B, carotenoids, mineral salts, amino acids, antioxidants, enzymes, probiotics, chocolate mixed with probiotics, oligoelements, animal extracts, vegetable extracts or their mixture.

More preferably, said additional active ingredients are selected from among β-carotene, n-acetylcysteine, glutathione, NADH, lipoic acid, coenzyme Q10, uric acid, melatonin, EDTA, flavonoids, resveratrol, yeast, ascorbyl palmitate, chitosan, alpha-tocopherol gamma-tocopherol, vitamins of group B (such as folic acid and B12), vitamin A, vitamin E, vitamin D, vitamin K, pantothenic acid, biotin, inositol, SAMe, calcium salts (preferably calcium carbonate), magnesium salts (preferably magnesium citrate), iron salts (preferably ferrous citrate), copper salts (preferably copper gluconate), iodine salts (preferably sodium iodine), zinc salts (preferably zinc citrate), manganese salts (preferably manganese carbonate), sodium salts (preferably sodium citrate), potassium salts (preferably potassium bicarbonate), selenium salts (preferably sodium selenite), chromium salts (preferably chromium sulphate), molybdenum salts (preferably sodium molybdate), fluorine salts (preferably sodium fluoride), chlorine salts (preferably potassium chloride), phosphorous salts (preferably potassium salts of orthophosphoric acid), soluble fibres (preferably inulin), gelling insoluble fibres (preferably glucomannan and/or guar gum), lactoferrin, lactulose, arabinogalactan, beta-glucan, green tea extract, chlorella pyrenoidosa, melilot, curcuma, pilocarpus jaborandi, centella asiatica, common dandelion, nux vomica, ES ephedra or a mixture thereof.

The compositions according to the present invention are coated with chocolate. Said chocolate is preferably milk or dark chocolate mixed with synthetic or natural sweeteners.

According to the present invention, the term chocolate preferably refers to a mixture of sugar and/or other sweeteners (such as for example fructose, maltitol, isomalt, sorbitol), cocoa powder (preferably about 11-20% of fat content), cocoa butter (preferably about 95% of fat content), cocoa paste (preferably about 50/55% of fat content) and soy lecithin. Milk chocolate is obtained by mixing dark chocolate with milk powder at various concentrations.

The term sweeteners is used to indicate synthetic or natural sweetening substances preferably selected from among, xylitol, maltitol, sorbitol, mannitol, sucralose, acesulfame K, sodium cyclamate, aspartame, sucrose, fructose, dextrose, maltose, isomalt, spray dried malt, sodium aspartate, neohesperidine, citrus extract or their mixture.

In the coated compositions according to the invention, part of the active ingredient may be directly dispersed into the coating itself and then be poured onto the final composition in form of tablet, granule or capsule. In particular, obtained according to this aspect of the invention are pastilles ("drageè") having an outer chocolate coating in which liposoluble, and non-liposoluble, active ingredients may be incorporated in a palatable manner.

The abovementioned coating is present in the compositions of the invention at an amount varying between 15% and 60%, preferably between 20% and 40%, with respect to the total weight of the composition.

Further object of the present invention are the abovementioned orobuccal compositions for use in the treatment, human and veterinary, of diseases or disorders of the eye, for example, cataract, glaucoma, senile maculopathy.

The abovementioned compositions are also for use in the protection the visual system against oxidative stress, improvement of visual functionality with respect to direct glare (reduction of the time required for recovery from direct glare stress), improvement of long distance sight as well as improving colour contrast.

The compositions of the present invention may be prepared by means of a process comprising the following steps:
Scheme A (for orobuccal tablets, granules or capsule):
   1) Weighing the active components and excipients;
   2) Mixing (mixer with rotating screw);
   3) Final compression or use of the mixture obtained in step 2 directly packaged in sachets or capsules.
Scheme B (for orobuccal tablets, granules or capsules):
   1) Weighing the active components and excipients;
   2) Mixing (mixer with rotating screw);
   3) Compression;
   4) Dry granulation of the tablets obtained by passing through an oscillating granulator;
   5) Final compression or use of the granules obtained in step 4 directly packaged in sachets or capsules.
Scheme C (for orobuccal tablets, granules or capsules):
   1) Mixing lutein, cocoa paste or other molten fat under heat;
   2) Granulation, after cooling the mixture and adding maltitol and/or other excipients;
   3) Final compression or use of the mixture obtained in step 2 directly packaged in sachets or capsules.

### Process of coating with chocolate

1) Melt the chocolate, the active ingredient and sweetener in water bath;
2) Pour the mixture obtained at point 1) onto the composition directly in the pan.
3) The coating is repeated with molten mixture and cooled several times;
4) The coated composition obtained at point 3) may be optionally polished using suitable excipients.

The following examples describe in detail the compositions of the invention.

### EXAMPLES

### Example 1 - according to the invention

### Orobuccal tablet based on lutein and zeaxanthin

- Mixture of lutein and zeaxanthin 75 mg (11.54%)
- Sodium bicarbonate 10 mg (1.54%)
- Maltitol 109 mg (16.77%)
- Sucrose 129 mg (19.84%)
- Chocolate flakes 184.18 mg (28.35%)
- Cocoa 130.55 mg (20.08%)
- Vanilla flavour 0.67 mg (0.10%)
- Microcrystalline cellulose 5.8 mg (0.89%)
- Silica gel 5.8 mg (0.89%)
- TOT: 650 mg (100%)

Orobuccal tablets are made weighing about 0.6 g, coated with chocolate.

The mixture of lutein and zeaxanthin corresponds to the microencapsulated form containing lutein 20% and zeaxanthin 0.8%, equivalent to 15 mg of lutein and 0.6 mg of zeaxanthin in free form.

### Example 2 - reference example, not according to the invention

### Sublingual tablet based on Lutein and Zeaxanthin

- Mixture of lutein and zeaxanthin 50 mg (20.83%)
- Sodium bicarbonate 10 mg (4.16%)
- Xylitol 115.7 mg (48.22%)
- Tween 80 5 mg (0.38%)
- Dried maize starch 50 mg (20.84%)
- Crospovidone 5 mg (2.08%)
- Silica gel 2.5 mg (1.04%)
- Magnesium stearate 1.6 mg (0.67%)
- Sucralose 0.2 mg (0.08%)
- TOT: 240 mg (100%)

Sublingual tablets are made weighing about 0.2 g.

The mixture of lutein and zeaxanthin corresponds to the microencapsulated form containing lutein 20% and zeaxanthin 0.8%, equivalent to 10 mg of lutein and 0.4 mg of zeaxanthin in free form.

### Example 3 - according to the invention

### Orobuccal tablet based on lutein and zeaxanthin

- Mixture of lutein and zeaxanthin 40 mg (3.08%)
- Soy lecithin 50 mg (3.85%)
- Tween 80 5 mg (0.38%)
- Sodium bicarbonate 15 mg (1.15%)
- Vit E 10 mg (0.77%)
- Resveratrol 5 mg (0.38%)
- Maltitol 470 mg (36.14%)
- Cocoa paste 215mg (16.53%)
- Citrus extract 2 mg (0.12%)
- Silica gel 3 mg (0.23%)
- TOT: 815 mg

Orobuccal tablets are made weighing about 0.8 g, coated - according to the method described above - using cocoa paste 450 mg (34.61%), 35 mg (2.69%) oil containing 20% lutein and 0.8% zeaxanthin (equivalent to 7 mg of lutein and 0.28 mg of zeaxanthin) and sucralose 1 mg (0.06%).

| | |
|---|---|
| Total of the coated tablet | 1300 mg (100%) |

Coated tablets are made weighing about 1.3 g
The mixture of lutein and zeaxanthin on which the tablet is based corresponds to the microencapsulated form containing lutein 20% and zeaxanthin 0.8%, equivalent to 8 mg of lutein and 0.32 mg of zeaxanthin in free form.

### Example 4 - reference example, not according to the invention

### Orobuccal tablet based on lutein and zeaxanthin

- Mixture of lutein and zeaxanthin 75 mg (6.15%)
- Silica gel 100 mg (8.19%)
- Tween 80 5 mg (0.40%)
- DHA, EPA 50 mg (4.10%)
- Ascorbic acid 60 mg (4.91%)
- Xylitol 500 mg (40.10%)
- Mannitol 300 mg (24.60%)
- Lyophilized orange 100 mg (8.19%)
- Orange flavour 20 mg (1.64%)
- Magnesium stearate 10 mg (0.82%)
- TOT: 1220 mg (100%)

Orobuccal tablets are made having a diameter of 16 mm and weighing about 1.2 g.

The mixture of lutein and zeaxanthin corresponds to the oil form containing lutein 20% and zeaxanthin 0.8%, equivalent to 15 mg of lutein and 0.6 mg of zeaxanthin in free form.

### Example 5 - reference example, not according to the invention

### Effervescent tablet based on lutein and zeaxanthin

- Mixture of lutein and zeaxanthin 150 mg (6.21 %)
- Tween 80 5 mg (0.20%)
- Tocopherol 50 mg (2.08%)
- N-acetylcysteine 50 mg (2.08%)
- Anhydrous sodium bicarbonate 400 mg (16.56%)
- Citric acid 250 mg (10.35%)
- Adipic acid 250 mg (10.35%)
- Sodium carbonate 100 mg (4.14%)
- Maltitol 900 mg (37.27%)
- Lime flavour 200 mg (8.28%)
- Silica gel 10 mg (0.40%)
- Leucine 50 mg (2.08%)
- TOT: 2415 mg (100%)

Orobuccal tablets are made having a diameter of 19 mm weighing about 2.4 g.

The mixture of lutein and zeaxanthin corresponds to the microencapsulated form containing lutein 20% and zeaxanthin 0.8%, equivalent to 15 mg of lutein and 0.6 mg of zeaxanthin in free form.

### Example 6 - according to the invention

### Orobuccal tablet based on lutein and zeaxanthin

- Mixture of lutein and zeaxanthin 75 mg (6.40%)
- Soy lecithin 50 mg (4.27%)
- Tween 80 5 mg (0.42%)
- Sodium bicarbonate 15 mg (1.28%)
- Lycopene 50 mg (4.27%)
- Lipoic acid 50 mg (4.27%)
- Maltitol 650 mg (55.31%)
- Cocoa powder 150 mg (12.80%)
- Cocoa paste 153.2 mg (12.80%)
- Sucralose 1.8 mg (0.31%)
- TOT: 1200 mg (100%)

Orobuccal tablets are made weighing about 1.2 g, coated with chocolate.

La mixture of lutein and zeaxanthin corresponds to the microencapsulated form containing lutein 20% and zeaxanthin 0.8%, equivalent to 15 mg of lutein and 0.6 mg of zeaxanthin in free form.

### Example 7 - reference example, not according to the invention

### Granules based on lutein and zeaxanthin

- Mixture of lutein and zeaxanthin 150 mg (11.30%)
- Soy lecithin 30 mg (2.50%)
- Tween 80 5 mg (0.42%)
- Sodium bicarbonate 15 mg (1.25%)
- Chocolate with probiotics 50 mg (4.16%)
- Mannitol 550 mg (45.78%)
- White cocoa paste 300 mg (24.97%)
- Low-fat milk 23.6 mg (12.48%)
- Sucralose 1.4 mg (0.12%)
- TOT: 1300 mg (100%)

Orobuccal granules are obtained packaged in sachets weighing about 1.3 g.

The mixture of lutein and zeaxanthin corresponds to the dispersible powder form containing lutein 10% and Zeaxanthin 0.4%, equivalent to 15 mg of lutein and 0.6 mg of zeaxanthin in free form.

### Example 8 - reference example, not according to the invention

### Orobuccal tablet based on lutein and zeaxanthin for veterinary use

- Mixture of lutein and zeaxanthin 75 mg (5.22%)
- Silica gel 50 mg (3.48%)
- Phospholipids 50 mg (3.48%)
- Calcium ascorbate 60 mg (4.18%)
- Glutathione 100 mg (6.98%)
- Yeast 700 mg (48.78%)
- Liver 400 mg (27.87%)
- TOT: 1435 mg (100%)

Orobuccal tablets are made having a diameter of 16 mm and weighing about 1.4 g.

The mixture of lutein and zeaxanthin corresponds to the microencapsulated form containing lutein 20% and zeaxanthin 0.8%, equivalent to 15 mg of lutein and 0.6 mg of zeaxanthin in free form.

### Example 9 - reference example, not according to the invention

### Orobuccal tablet based on lutein and zeaxanthin for veterinary use

- Mixture of lutein and zeaxanthin 150 mg (13.88%)
- Silica gel 50 mg (4.63%)
- Soy lecithin 50 mg (4.63%)
- Calcium ascorbate 60 mg (5.55%)
- Glutathione 100 mg (9.27%)
- NADH 10 mg (0.93%)
- Yeast 670 mg (60.13%)
- Magnesium stearate 10 mg (0.93%)
- TOT: 1100 mg (100%)

Orobuccal tablets are made having a diameter of 16 mm and weighing about 1.1 g.

The mixture of lutein and zeaxanthin corresponds to the dispersible powder form containing lutein 10% and zeaxanthin 0.4%, equivalent to 15 mg of lutein and 0.6 mg of zeaxanthin in free form.

### Example 10 - reference example, not according to the invention

### Orobuccal granules based on lutein and zeaxanthin for veterinary use

- Mixture of lutein and zeaxanthin 75 mg (4.73%)
- Silica gel 50 mg (3.16%)
- Phospholipids 50 mg (3.16%)
- Calcium ascorbate 60 mg (3.78%)
- Glutathione 100 mg (6.31%)
- Yeast 700 mg (44.16%)
- Liver 415 mg (25.24%)
- TOT: 1600 mg (100%)

Orobuccal granules are obtained packaged in sachets weighing about 1.6 g.

The mixture of lutein and zeaxanthin corresponds to the microencapsulated form containing lutein 20% and zeaxanthin 0.8%, equivalent to 15 mg of lutein and 0.6 mg of zeaxanthin in free form.

### Example 11 - reference example, not according to the invention

### Slightly effervescent sublingual tablets based on lutein

- Mixture of lutein and zeaxanthin 75 mg (21.43%)
- Sodium bicarbonate 100 mg (28.57%)
- Citric acid 50 mg (14.28%)
- Lyophilized orange 56 mg (16.00%)
- Magnesium stearate 3 mg (0.86%)
- Silica gel 10 mg (2.86%)
- Maize starch 50 mg (14.28%)
- Orange flavour 2 mg (0.57%)
- Crospovidone 3 mg (0.86%)
- Sucralose 1 mg (0.29%)
- TOT: 350 mg (100%)

Slightly effervescent sublingual tablets are obtained weighing about 0.35 g.
The mixture of lutein and zeaxanthin corresponds to the microencapsulated form containing lutein 20% and zeaxanthin 0.8%, equivalent to 15 mg of lutein and 0.6 mg of zeaxanthin in free form.

### Example 12 - according to the invention

### Orobuccal pastille (drageè) coated with chocolate based on lutein and zeaxanthin

- Mixture of lutein and zeaxanthin 40 mg (2.89 %)
- Soy lecithin 50 mg (3.6%)
- Tween 80 5 mg (0.36%)
- Sodium bicarbonate 15 mg (1.08%)
- Vit E 10 mg (0.72%)
- Resveratrol 5 mg (0.36%)
- Vita A 1200 mcg (0.08%)
- Vit D 7.5 mcg (0.005%)
- Vit K 105 mcg (0.007%)
- Calcium ascorbate 250 mg (18.03%)
- Sodium fluoride 4 mg (0.29%)
- Maltitol 299.62 mg (21.61 %)
- Cocoa paste 215 mg (15.51%)
- Citrus extract 2 mg (0.14%)
- Silica gel 3 mg (0.21%)
- TOT: 900 mg (64.93%)

Orobuccal pastilles (drageè) are made weighing about 0.9 g, coated according to the method described above using:
- Chocolate 450 mg (32.46%),
- Mixture of lutein and zeaxanthin 35 mg (0.25%)
- Sucralose 1 mg (0.07%).

| | |
|---|---|
| Total coating with chocolate | 486 mg (35.07%) |
| Total of the coated tablet | 1386 mg (100%) |

Coated tablets are made weighing about 1.38 g.

The mixture of lutein and zeaxanthin on which the tablet is based corresponds to the microencapsulated form containing lutein 20% and zeaxanthin 0.8%, equivalent to 8 mg of lutein and 0.32 mg of zeaxanthin in free form.

## Claims

1. A pharmaceutical, dietary and/or nutraceutical orobuccal composition in form of tablet, capsule, granules or pastille containing a mixture of lutein and zeaxanthin together with at least one physiologically acceptable excipient, **characterised in that** it comprises an outer coating containing chocolate.

2. Composition according to claim 1, **characterised in that** said outer coating is chocolate.

3. Composition according to claim 1, **characterised in that** part of the mixture of lutein and zeaxanthin is dispersed in said outer coating.

4. Composition according to claim 1, wherein said at least one physiologically acceptable excipient is selected from among emulsifiers, surfactants or a mixture thereof.

5. Composition according to claim 4, wherein said emulsifier is soy lecithin.

6. Composition according to claim 4, wherein said surfactant is selected from among polysorbate 80, sodium lauryl sulphate or a mixture thereof.

7. Composition according to claim 4, wherein said at least one excipient is a mixture of polysorbate 80 and soy lecithin.

8. Composition according to claim 1, wherein said lutein is (3R, 3'R, 6'R)-β,ε-carotene-3,3'-diol and said zeaxanthin is (3R, 3'R)-β,β-carotene-3,3'-diol.

9. Composition according to claim 1, wherein said mixture of lutein and zeaxanthin is present at a amount varying between 0.1 and 5% in weight, preferably between 0.15% and 4.5% in weight, with respect to the total weight of the composition.

10. Composition according to claim 1, comprising at least one further active ingredient.

11. Composition according to claim 10, wherein said at least one further active ingredient is present at an amount varying between 1% and 45% in weight, preferably between 1.5% and 40% in weight, with respect to the total weight of the composition

12. Composition according to claim 10, wherein said at least one further active ingredient is selected from among short chain fatty acids, medium chain fatty acids, vitamin C, vitamin D, vitamins of group B, carotenoids, mineral salts, amino acids, antioxidants, enzymes, probiotics, chocolate mixed with probiotics, oligoelements, animal extracts, vegetable extracts or a mixture thereof.

13. Composition according to claim 1, in form of effervescent tablet.

14. Composition according to claim 1, in form of effervescent granules.

15. Composition according to claim 1, formulated in sublingual form.

16. Composition according to claim 15, in form of sublingual tablet or sublingual effervescent tablet.

17. Composition according to claim 15, in form of sublingual granules or sublingual effervescent granules.

18. Composition according to claim 1, in form of pastille.

19. Composition according to claim 1, **characterised in that** it has high palatability.

20. Composition according to any one of the preceding claims, for use in the treatment of diseases and/or disorders of the eye.

21. Composition according to claim 20, for use in the treatment of cataract, glaucoma and/or senile maculopathy.

22. Composition according to any one of the preceding claims, for use in the protection of the visual system against oxidative stress, in the improvement of visual functionality with respect to direct glare light, improvement of long-distance sight and/or improvement of colour contrast.

23. Composition according to any one of the preceding claims, for use in human or veterinary treatment.

## Patentansprüche

1. Pharmazeutische, Nahrungs- und/oder Funktions- orobukkale Zusammensetzung in Form einer Tablette, Kapsel, eines Granulats oder einer Pastille, die ein Gemisch von Lutein und Zeaxanthin zusammen mit mindestens einem physiologisch annehmbaren Exzipienten enthält, **dadurch gekennzeichnet, dass** sie eine äußere Schicht mit Schokolade enthält.

2. Zusammensetzung nach Anspruch 1, **dadurch gekennzeichnet, dass** die äußere Schicht Schokolade ist.

3. Zusammensetzung nach Anspruch 1, **dadurch gekennzeichnet, dass** ein Teil des Gemisches von Lutein und Zeaxanthin in der äußeren Schicht dispergiert ist.

4. Zusammensetzung nach Anspruch 1, worin der mindestens eine physiologisch annehmbare Exzipient ausgewählt ist unter Emulgatoren, oberflächenaktiven Mitteln oder einem Gemisch davon.

5. Zusammensetzung nach Anspruch 4, worin der Emulgator Sojalecithin ist.

6. Zusammensetzung nach Anspruch 4, worin das oberflächenaktive Mittel ausgewählt ist unter Polysorbat 80, Natriumlaurylsulfat oder einem Gemisch davon.

7. Zusammensetzung nach Anspruch 4, worin der mindestens eine Exzipient ein Gemisch von Polysorbat 80 und Sojalecithin ist.

8. Zusammensetzung nach Anspruch 1, worin das Lutein (3R, 3'R, 6'R)-β,ε-Caroten-3,3'-diol und das Zeaxanthin (3R, 3'R)-β,β-Caroten-3,3'-diol ist.

9. Zusammensetzung nach Anspruch 1, worin das Gemisch von Lutein und Zeaxanthin in einer Menge vorhanden ist von zwischen 0,1 und 5 Gew.-%, vorzugsweise zwischen 0,15 Gew.-% und 4,5 Gew.-%, bezogen auf das Gesamtgewicht der Zusammensetzung.

10. Zusammensetzung nach Anspruch 1, welches mindestens einen weiteren aktiven Inhaltsstoff umfasst.

11. Zusammensetzung nach Anspruch 10, worin der mindestens eine weitere Inhaltsstoff in einer Menge vorhanden ist von zwischen 1 Gew.-% und 45 Gew.-%, vorzugsweise zwischen 1,5 Gew.-% und 40 Gew.-%, bezogen auf das Gesamtgewicht der Zusammensetzung.

12. Zusammensetzung nach Anspruch 10, worin der mindestens eine weitere aktive Inhaltsstoff ausgewählt ist unter kurzkettigen Fettsäuren, Fettsäuren mit mittlerer Kettenlänge, Vitamin C, Vitamin D, Vitaminen der Gruppe B, Karotenoiden, Mineralsalzen, Aminosäuren, Antioxidantien, Enzymen, Probiotika, Schokolade vermischt mit Probiotika, Oligoelementen, Tierextrakten, Gemüseextrakten oder einem Gemisch davon.

13. Zusammensetzung nach Anspruch 1, in Form einer Brausetablette.

14. Zusammensetzung nach Anspruch 1, in Form von Brause-Granulaten.

15. Zusammensetzung nach Anspruch 1, formuliert in sublingualer Form.

16. Zusammensetzung nach Anspruch 15, in Form einer sublingualen Tablette oder sublingualen Brause-Tablette.

17. Zusammensetzung nach Anspruch 15, in Form von sublingualen Granulaten oder sublingualen Brause-Granulaten.

18. Zusammensetzung nach Anspruch 1, in Form einer Pastille.

19. Zusammensetzung nach Anspruch 1, **dadurch gekennzeichnet, dass** sie eine hohe Schmackhaftigkeit aufweist.

20. Zusammensetzung nach einem der vorstehenden Ansprüche, zur Verwendung bei der Behandlung von Erkrankungen und/oder Störungen des Auges.

21. Zusammensetzung nach Anspruch 20 zur Verwendung bei der Behandlung von Katarakt, Glaulcom und/oder Alters-Makulopathie.

22. Zusammensetzung nach einem der vorstehenden Ansprüche, zur Verwendung beim Schutz des visuellen Systems gegen oxidativen Stress, bei der Verbesserung der visuellen Funktionalität in Bezug auf direktes Blendlicht, bei der Verbesserung der Fernsicht und/oder Verbesserung des Farbkontrasts.

23. Zusammensetzung nach einem der vorstehenden Ansprüche, zur Verwendung bei der Behandlung von Menschen und Tieren.

## Revendications

1. Composition pharmaceutique, nutritionnelle et/ou nutraceutique à prendre *per os* sous forme de comprimé, capsule, granules ou pastille contenant un mélange de lutéine et de zéaxanthine conjointement avec au moins un excipient physiologiquement acceptable, **caractérisée en ce qu'**elle comprend un enrobage contenant du chocolat.

2. Composition selon la revendication 1, **caractérisée en ce que** ledit enrobage est du chocolat.

3. Composition selon la revendication 1, **caractérisée en ce qu'**une partie du mélange de lutéine et de zéaxanthine est dispersée dans ledit enrobage.

4. Composition selon la revendication 1, dans laquelle ledit au moins un excipient physiologiquement acceptable est choisi parmi les agents émulsifiants, les tensioactifs ou un mélange de ceux-ci.

5. Composition selon la revendication 4, dans laquelle ledit agent émulsifiant est de la lécithine de soja.

6. Composition selon la revendication 4, dans laquelle ledit tensioactif est choisi parmi le polysorbate 80, le laurylsulfate de sodium ou un mélange de ceux-ci.

7. Composition selon la revendication 4, dans laquelle ledit au moins un excipient est un mélange de polysorbate 80 et de lécithine de soja.

8. Composition selon la revendication 1, dans laquelle ladite lutéine est du (3R, 3'R, 6'R)-β,ε-carotène-3,3'-diol et ladite zéaxanthine est du (3R, 3'R)-β,β-carotène-3,3'-diol.

9. Composition selon la revendication 1, dans laquelle ledit mélange de lutéine et de zéaxanthine est présent en une quantité variant entre 0,1 et 5 % en poids, de préférence entre 0,15 % et 4,5 % en poids, par rapport au poids total de la composition.

10. Composition selon la revendication 1, comprenant au moins une autre substance active.

11. Composition selon la revendication 10, dans laquelle ladite au moins une autre substance active est présente en une quantité variant entre 1 % et 45 % en poids, de préférence entre 1,5 % et 40 % en poids, par rapport au poids total de la composition

12. Composition selon la revendication 10, dans laquelle ladite au moins une autre substance active est choisie parmi les acides gras à chaîne courte, les acides gras à chaîne moyenne, la vitamine C, la vitamine D, les vitamines du groupe B, les caroténoïdes, les sels minéraux, les acides aminés, les antioxydants, les enzymes, les probiotiques, le chocolat mélangé avec des probiotiques, les oligoéléments, les extraits d'origine animale, les extraits d'origine végétale ou un mélange de ceux-ci.

13. Composition selon la revendication 1, sous forme de comprimé effervescent.

14. Composition selon la revendication 1, sous forme de granules effervescents.

15. Composition selon la revendication 1, préparée sous une forme sublinguale.

16. Composition selon la revendication 15, sous forme de comprimé à prendre par voie sublinguale ou de comprimé effervescent à prendre par voie sublinguale.

17. Composition selon la revendication 15, sous forme de granules à prendre par voie sublinguale ou de granules effervescents à prendre par voie sublinguale.

18. Composition selon la revendication 1, sous forme de pastille.

19. Composition selon la revendication 1, **caractérisée en ce qu'**elle a une palatabilité élevée.

20. Composition selon l'une quelconque des revendications précédentes, pour une utilisation dans le traitement de maladies et/ou de troubles de l'oeil.

21. Composition selon la revendication 20, pour une utilisation dans le traitement de la cataracte, du glaucome et/ou de la maculopathie sénile.

22. Composition selon l'une quelconque des revendications précédentes, pour une utilisation dans la protection du système visuel contre le stress oxydatif, dans l'amélioration de la fonctionnalité visuelle par rapport à la lumière éblouissante directe, l'amélioration de la vision de loin et/ou l'amélioration du contraste des couleurs.

23. Composition selon l'une quelconque des revendications précédentes, pour une utilisation dans le traitement humain ou vétérinaire.
